# EUROPEAN PATENT APPLICATION

(11) **EP 1 027 900 A1**
(43) Date of publication of application: **16.08.2000**
(21) Application number: 99200379.8
(22) Date of filing: 09.02.1999
(51) Int. Cl.: A61M 5/14, A61J 15/00

(54) **Method and apparatus for hydrating by enteral delivery**

(71) Applicant: SOCIETE DES PRODUITS NESTLE S.A., 1800 Vevey (CH)
(72) Inventor: Larrain, Ignaccio, 1028 Preverenges (CH); Bourguignon, Michel, 14740 Lasson (FR)

(57) **Abstract**

An enteral set which enables the sterile hydration of enterally fed patients. The set is made up of an enteral spike for spiking a containing, a sealable inlet port, and a tube set for delivery to a patient of liquid received from the enteral spike. In use, liquids may flow from the container through the enteral spike to the tube set. Also a hydration liquid may be introduced through the sealable inlet port to flow through the enteral spike and into the container. The hydration liquid may then be drained from the container in the normal manner.

## Description

### Field of the Invention

This invention relates to a method for hydrating a patient in conjunction with enteral feeding. The invention also relates to a device which enables the hydration of patients during enteral feeding.

### Background to the Invention

In clinical settings, feeding solutions are commonly tube-fed to patients. The feeding solution is held in a container from whence it is dispensed to the patient through a tubing set; either using gravity or a pump. The tubing set is normally made up of tubing having an inlet connector at one end and a outlet connector at the other end. The inlet connector is connected to the container to create a flow path for the feeding solution into the tubing. The outlet connector is connected to suitable device for introducing the feeding solution into the patient. For example, in enteral feeding, the device is usually a naso-gastric feeding tube. The tubing set may also have various other devices connected in it or to it; for example a drip chamber, a clamp, a Y-connector, and the like.

Apart from feeding the feeding solution, it is also common to hydrate the patient by providing water in one form or another. For feeding solutions which are held in open containers or in bottles, this is often done by pouring water or tea into the bottle or container. The water or tea then flows through the tubing set to the patient. Usually, the water or tea is added to the container after the feeding solution has been fed to the patient. However, it can also be added during feeding of the feeding solution although this is not generally recommended.

In recent times, there has been a move away from using bottles and opened topped containers to using flexible bags. An example of a suitable bag is disclosed in US patent 5259844. When bags are used, the inlet connector takes the form of a spike which pierces a seal in the outlet port of the bag. The bags offer the advantage that they are a closed, sterile system. Therefore, the possibility of the feeding solution being contaminated during feeding is reduced.

However, precisely because the bags are a closed system, hydrating the patient becomes more difficult. In certain cases, the staff caring for the patient have even been known to cut a hole into the bag to add water or tea to the bag. This of course removes the primary advantage of the bag.

Therefore, there is a need for method and apparatus which enables the hydration of patients who are being fed feeding solutions held in feeding bags.

### Summary of the Invention

According, in one aspect, this invention provides a spike connector enabling hydration of patients being enterally fed, the spike connector comprising:-
an elongate body which has a channel through it from a distal end to a tube end and through which liquids may flow, the channel having an inlet at the distal end and an outlet at the tube end, the elongate body being formed into a spike at the distal end for creating liquid connection with a container for a feeding solution; and
a sealable inlet port connecting to the channel of the elongate body;
whereby feeding solution may flow from the feeding bag through the elongate body and out of the outlet and a hydration liquid may be introduced through the inlet port and into the container.

Preferably a locking member is positioned on the elongate body for locking with the container.

In one embodiment, the sealable inlet port forms part of a Y-connector which is attachable to the tube end of the elongate body. In another embodiment, the sealable inlet port forms part of a 3-way valve which is attachable to the tube end of the elongate body. In yet another embodiment, the sealable inlet port is an integral part of the elongate body.

In another aspect, this invention provides an enteral set enabling hydration of enterally fed patients, the set comprising:-
an enteral spike for creating liquid communication with a container and permitting flow of liquids from the container through the enteral spike;
a sealable inlet port for introducing a hydration liquid into the enteral spike; and
a tube set for delivery to a patient of liquid received from the enteral spike;
whereby liquids may flow from the container through the enteral spike to the tube set, and a hydration liquid may be introduced through the sealable inlet port to flow through the enteral spike and into the container.

In a yet another aspect, this invention provides a method for providing hydration liquid to a tube fed patient in a sterile manner, the method comprising:-
providing an enteral set which includes an enteral spike; a sealable inlet port for introducing liquids into the enteral spike; and a tube set for delivery to a patient of liquid received from the enteral spike;
connecting the enteral spike to a liquid container containing a feeding solution and allowing the feeding solution to flow from the liquid container through the enteral set;
introducing a hydration liquid into the enteral spike through the sealable inlet port and causing the hydration liquid to flow into the container; and
sealing the sealable inlet port and allowing the hydration liquid to flow from the liquid container through the enteral set.

Preferably, the hydration liquid is introduced into the enteral spike after the feeding solution has substantially completely drained from the container.

### Brief Description of the Drawing

Embodiments of the invention are now described, by way of example only, with reference to the drawings in which:

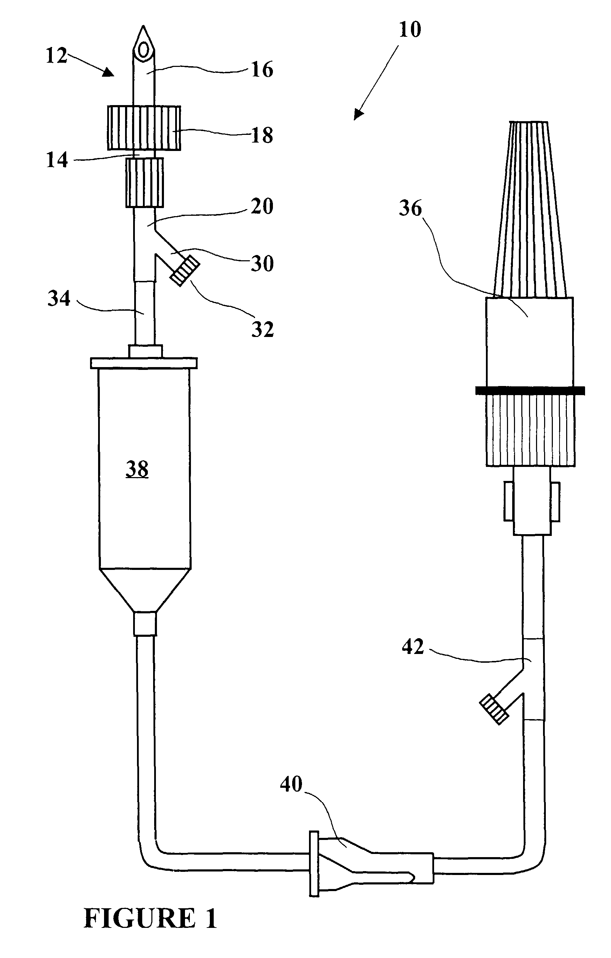

Figure 1 is a schematic illustration of an enteral set; and

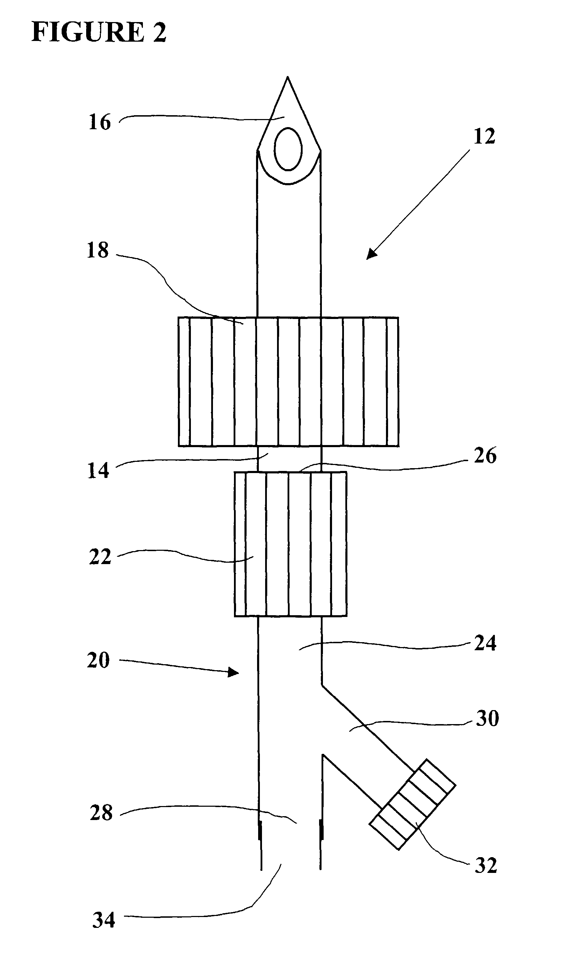

Figure 2 is a schematic illustration of the spike of the enteral set of Figure 1.

### Detailed Description of the Preferred Embodiments

The drawings illustrate an enteral set 10 which enables hydration of patients who are being fed from flexible bags. The enteral set 10 has a spike 12 at one end. The spike 12 may be a standard enteral spike or may be designed as desired. The spike has an elongate body 14 which has a channel through it through which feeding solution may flow. The distal end of the spike 12 has a sharp tip 16 for piercing the seal in the outlet port of a flexible bag (not shown). A female revolving lock 18 is positioned about the elongate body 14. In use, the revolving lock 18 threads over the outlet port of the flexible bag. In this way, a substantially fluid tight join may be created between the spike 12 and the outlet port. Ordinarily, the revolving lock 18 is in the form of a cap having an internal thread. One example of a suitable spike is described in US patent 5735841.

A Y-connector 20 is connected to the opposite end of the spike 12; either directly by means of a lock 22 as shown or through a short piece of tubing. The Y-connector 20 may be a standard Y-connector. The Y-connector 20 which has a channel 24 through it defining an inlet 26 and an outlet 28. The Y-connector 20 also has an inlet port 30 leading to the channel 24. The distal end of the inlet port 30 terminates in a suitable connector such as a female Luer lock, Luer activated site, or conical syringe connection. A cap 32 is connected over the connector to close the inlet port 30. For best flow characteristics, the Y-connector 20 is preferably arranged such that the inlet port 30 is directed towards the inlet 26 of the Y-connector 20.

Tubing 34 is connected to the outlet 28 of the Y-connector 20. The tubing 34 terminates in a terminal connector 36 which may be connected to a suitable device for introducing feeding solution into the patient; for example, a naso-gastric feeding tube. A drip chamber 38 is connected into the tubing 34 near the Y-connector 20. Further, a flow clamp 40 is connected to the tubing 34 to regulate the liquid flow rate through the tubing 34. An additional Y-connector 42 may be connected into the tubing 34 near the terminal connector 36. The additional Y-connector 42 permits the introduction of wash liquids.

In use, the spike 12 is pushed into the outlet port of a flexible bag to create a liquid connection between the bag and the spike 12. The revolving lock 18 is threaded on the outlet port to lock the spike 12 to the outlet port. The feeding solution then drains through the spike 12, the Y-connector 20, the tubing 34 and the terminal connector 36 to the patient. The cap 32 is on the inlet port 30 of the Y-connector 20 and hence the feeding solution cannot flow out of the inlet port 30.

Once the bag has emptied, the cap 32 of the Y-connector 20 is removed. A suitable hydration syringe (not shown) is then connected to the connector at the distal end of the inlet port 30 of the Y-connector 20. The syringe contains hydration liquid. The syringe is activated to force the hydration liquid into the enteral set 10 and into the flexible bag. If the clamp 40 is closed prior to introduction of the hydration liquid, the major portion of the hydration liquid flows into the flexible bag. The syringe may then be withdrawn and the cap 32 replaced on the inlet port 30 to seal it. Of course, the syringe may be left connected to the inlet port 30 and need not be removed. If the clamp 40 had been previously closed, it is then opened to allow the hydration liquid to drain through the enteral set to the patient. In this way, the patient is provided with hydration liquid without seriously compromising the sterility of the system.

It will be appreciated that any suitable means of introducing the hydration liquid into the inlet port 30 of the Y-connector 20 may be used; it is not necessary to use a syringe.

It will also be appreciated that numerous modifications may be made to the enteral set described above. For example, the spike 12 and the Y-connector 20 may be integrally formed into one piece. In this case, the spike has an inlet port extending into it; for example beneath the revolving lock 18. Such a spike may be manufactured by any suitable means such as injection moulding.

As an example of another modification, the Y-connector 20 may be replaced by a 3-way valve.

Further, while the enteral set has been described as it would be used for gravity feeding of a patient, the enteral set may be readily adapted for pump feeding of the patient. This may be done by connecting a suitable enteral pump into the tubing 34.

It is also not necessary for the feeding solution to be held in a flexible bag. For example, the spike 12 may be used with containers such as bottles and the like. For some containers, a suitable adapter may need to be connected between the spike 12 and the container. These adapters are commercially available.

## Claims

1. A spike connector enabling hydration of patients being enterally fed, the spike connector comprising:-
an elongate body which has a channel through it from a distal end to a tube end and through which liquids may flow, the channel having an inlet at the distal end and an outlet at the tube end, the elongate body being formed into a spike at the distal end for creating liquid connection with a container for a feeding solution; and
a sealable inlet port connecting to the channel of the elongate body;
whereby liquids may flow from the feeding bag through the elongate body and out of the outlet and a hydration liquid may be introduced through the inlet port and into the container.

2. A connector according to claim 1 in which the sealable inlet port is provided by a Y-connector at the tube end of the elongate body.

3. A connector according to claim 2 in which the Y-connector is directly connected to the elongate body.

4. A connector according to claim 1 in which the sealable inlet port is provided by a 3-way valve at the tube end of the elongate body.

5. A connector according to claim 1 in which the sealable inlet port is formed integrally in the elongate body.

6. An enteral set enabling hydration of enterally fed patients, the set comprising:-
an enteral spike for creating liquid communication with a container and permitting flow of liquids from the container through the enteral spike;
a sealable inlet port for introducing hydration liquid into the enteral spike; and
a tube set for delivery to a patient of liquid received from the enteral spike;
whereby liquids may flow from the container through the enteral spike to the tube set, and a hydration liquid may be introduced through the sealable inlet port to flow through the enteral spike and into the container.

7. An enteral set according to claim 6 in which a Y-connector is attached to the enteral spike between the enteral spike and the tube set, the Y-connector providing the sealable inlet port.

8. An enteral set according to claim 6 in which the sealable inlet port is formed integrally in the enteral spike.

9. A method for providing hydration liquid to a tube fed patient in a sterile manner, the method comprising:-
providing an enteral set which includes an enteral spike; a sealable inlet port for introducing liquids into the enteral spike; and a tube set for delivery to a patient of liquid received from the enteral spike;
connecting the enteral spike to a liquid container containing a feeding solution and allowing the feeding solution to flow from the liquid container through the enteral set;
introducing a hydration liquid into the enteral spike through the sealable inlet port and causing the hydration liquid to flow into the container; and
sealing the sealable inlet port and allowing the hydration liquid to flow from the liquid container through the enteral set.

10. A method according to claim 1 in which the hydration liquid is introduced into the enteral spike after the feeding solution has substantially completely drained from the container.
